# EUROPEAN PATENT APPLICATION

(11) **EP 4 467 127 A1**
(43) Date of publication of application: **27.11.2024**
(21) Application number: 23775372.8
(22) Date of filing: 20.03.2023
(51) Int. Cl.: A61K 9/00, A61K 47/26, A61K 47/02, A61K 31/4725, A61P 19/02, A61P 29/00

(54) **INJECTABLE PREPARATION COMPRISING ISOXAZOLINE DERIVATIVE, AND PREPARATION METHOD THEREFOR**

(30) Priority: 24.03.2022 KR 20220036549
(71) Applicant: LG Chem, Ltd., Yeongdeungpo-gu Seoul 07336 (KR)
(72) Inventor: BAEK, Jaeuk, Daejeon 34122 (KR); KIM, Sung Won, Daejeon 34122 (KR); YOON, Jung Woon, Daejeon 34122 (KR)
(74) Representative: Hoffmann Eitle
(86) International application number: PCT/KR2023/095013
(87) International publication number: WO 2023/182877

(57) **Abstract**

The present invention relates to a preparation for injection including an isoxazoline derivative useful as a caspase inhibitor or a pharmaceutically acceptable salt thereof as an active pharmaceutical ingredient (API).

The preparation for injection according to the present invention includes the first solution composed of a high-dose API and the second solution including a reconstitution solution, so that it can be prepared by mixing the first formulation and the second formulation immediately before administration to a patient, and contains the Active-API stably. Therefore, there is an advantage that effective drug efficacy can be expected when administered to a patient.

## Description

### [Technical Field]

This application claims the benefit of priority based on Korean Patent Application No. 10-2022-0036549 filed on March 24, 2022, all contents of which are incorporated as part of this specification.

The present invention relates to a preparation for injection including an isoxazoline derivative useful as a caspase inhibitor or a pharmaceutically acceptable salt thereof as an active pharmaceutical ingredient (API) and a method for manufacturing thereof.

### [Background Art]

Caspase is a cysteine protease, and a caspase inhibitor is a compound that can control inflammation or apoptosis caused by the action of caspase by inhibiting the action of this caspase. Diseases for which this compound can be administered to eliminate or alleviate symptoms include osteoarthritis, rheumatoid arthritis, and degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes, gastric ulcer and the like.

As caspase inhibitors, various types of isoxazoline derivatives and prodrugs of the isoxazoline derivatives are known. Among the isoxazoline derivatives, (R)-N-((2S,3S)-2-(fluoromethyl)-2-hydroxy-5-oxotetrahydrofuran-3-yl)-5-isopropyl-3-(isoquinolin-1-yl)-4,5-dihydroisoxazol-5-carboxamide (Hereinafter, referred to as "Compound of Formula 1") has already proven its efficacy in liver disease caused by hepatitis virus, liver fibrosis, and nonalcoholic steatohepatitis (NASH).

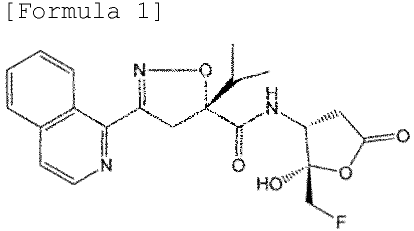

Recently, attempts have been made to apply the compound of Formula 1 to diseases related to bone and joint, such as osteoarthritis (OA), rheumatoid arthritis, degenerative arthritis, and destructive bone disorders.

In the treatment of the diseases related to bone and joint, a common drug administration method is to inject directly with a syringe into the joint cavity filled with synovial fluid. In the case of oral administration, the drug has a systemic effect, but systemic side effects are unavoidable. On the other hand, in the case of direct administration into the joint cavity, there are advantages in that a high concentration of the drug can be administered and systemic side effects can be minimized as well as a local therapeutic effect in which the drug is directly delivered due to local administration of the drug.

The compound of Formula 1 has been confirmed to have a therapeutic effect as the existing oral preparation for liver disease, but since the compound of Formula 1 is poorly soluble in water, it is difficult to formulate it in a preparation for injection.

Therefore, it is necessary to develop a high-concentration preparation for injection that can use the compound of Formula 1 as an injection.

### [Prior Art Documents]

### [Patent Document]

(Patent Document 1) Korean Patent No. 0774999(2007.11.02.) Isoxazoline derivatives and novel process for its preparation
(Patent Document 2) Korean Patent Application No. 2021-0102476(2021.08.04.) Use of caspase inhibitor for alleviating or treating osteoarthritis

### [Non-Patent Document]

(Non-Patent Document 1) Ratziu et al., 'A Phase 2, Randomized, Double-Blind, Placebo-Controlled Study of GS-9450 in Subjects with Nonalcoholic Steatohepatitis' HEPATOLOGY, Vol. 55, No. 2, 2012.

### [Disclosure]

### [Technical Problem]

Accordingly, the inventors of the present invention have conducted various studies to solve the above problems, and as a result, the problem to be solved by the present invention is to derive a preparation for injection including a high content of the compound of Formula 1 as an active pharmaceutical ingredient (API) .

In addition, in a preparation for injection including a high content of the compound of Formula 1 as an active pharmaceutical ingredient (API), it is an object to be solved by the present invention to derive a freeze-dried preparation for injection capable of increasing the stability of the active pharmaceutical ingredient and allowing long-term storage.

### [Technical Solution]

The present invention provides a method for manufacturing a preparation for injection comprising the steps of: (S1) preparing a first solution by dissolving the compound of Formula 1 below in a solvent including sugars;

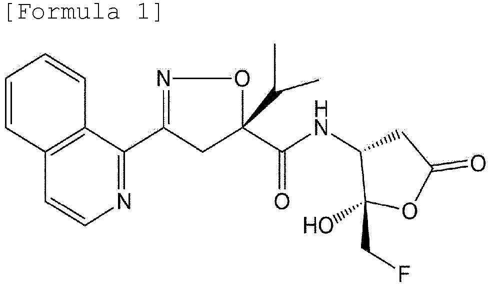

(S2) preparing a first formulation in the powder form by freeze-drying the first solution; and (S3) preparing a second formulation that is a reconstitution solution including a phosphate buffer, a surfactant, a pH adjusting agent, or a combination thereof.

Further, the present invention provides the method for manufacturing a preparation for injection wherein the pH of the solvent is 1 or lower.

Further, the present invention provides the method for manufacturing a preparation for injection wherein the solvent comprises hydrochloric acid, perchloric acid, nitric acid or sulfuric acid.

Further, the present invention provides the method for manufacturing a preparation for injection wherein the solvent comprises dimethyl sulfoxide or tert-butyl alcohol.

Further, the present invention provides the method for manufacturing a preparation for injection wherein the sugars are at least one selected from the group consisting of mannitol, lactose, sucrose, trehalose and sorbitol.

Further, the present invention provides the method for manufacturing a preparation for injection wherein the content of the sugars is 1 to 10%(w/w) based on the solvent.

Further, the present invention provides the method for manufacturing a preparation for injection wherein the surfactant is at least one selected from the group consisting of polysorbate 20, polysorbate 40, polysorbate 60, polysorbate 65 and polysorbate 80.

Further, the present invention provides the method for manufacturing a preparation for injection wherein the pH adjusting agent contained in the second formulation is NaOH.

The present invention provides a preparation for injection comprising: a first formulation in the powder form including the compound of Formula 1 below and sugars; and a second formulation that is a reconstitution solution including a phosphate buffer, a surfactant, a pH adjusting agent or a combination thereof:

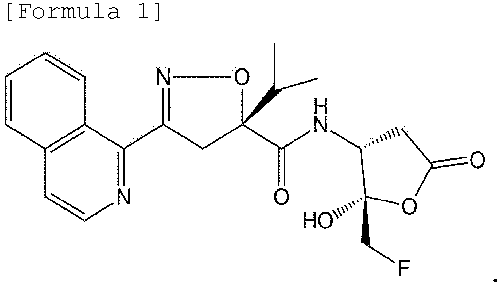

Further, the present invention provides the preparation for injection wherein the sugars are at least one selected from the group consisting of mannitol, lactose, sucrose, trehalose and sorbitol.

Further, the present invention provides the preparation for injection wherein the content of the sugars is 1 to 10%(w/w) based on the solvent.

Further, the present invention provides the preparation for injection wherein the surfactant is at least one selected from the group consisting of polysorbate 20, polysorbate 40, polysorbate 60, polysorbate 65 and polysorbate 80.

Further, the present invention provides the preparation for injection wherein the pH adjusting agent contained in the second formulation is NaOH.

Further, the present invention provides the preparation for injection wherein the content of the compound of Formula 1 is 1 to 20 mg/ml based on the mixed solution of the first formulation and the second formulation.

Further, the present invention provides a preparation for injection wherein the preparation for injection is for treating or preventing a disease selected from the group consisting of osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes and gastric ulcer.

Further, the present invention provides a preparation for injection wherein the disease is selected from the group consisting of osteoarthritis, rheumatoid arthritis, degenerative arthritis and destructive bone disorder.

Further, the present invention provides a preparation for injection which is used as a single-dose in a solution state in which the first formulation and the second formulation are mixed.

Further, the present invention provides a preparation for injection which is administered into the joint cavity after mixing the first formulation and the second formulation.

Further, the present invention provides a preparation for injection which is administered into the joint cavity within 30 minutes after mixing the first formulation and the second formulation.

Further, the present invention provides a preparation for injection which is administered once after mixing the first formulation and the second formulation.

The present invention provides a preparation for injection comprising: a first formulation in the powder form including the compound of Formula 1 below and sugars; and a second formulation that is a reconstitution solution including a phosphate buffer, a surfactant, a pH adjusting agent or a combination thereof, 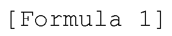 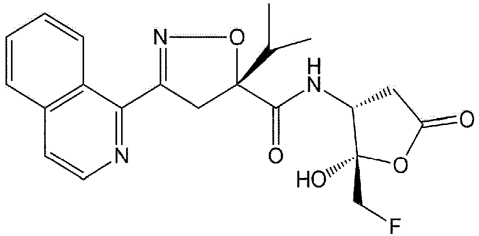 wherein, when the contents of the compounds of Formula 2 and Formula 3 below were measured within 30 minutes after mixing the first formulation and the second formulation, of the total contents of the compounds of Formula 2 and Formula 3, the content of the compound Formula 2 is 94% or more:

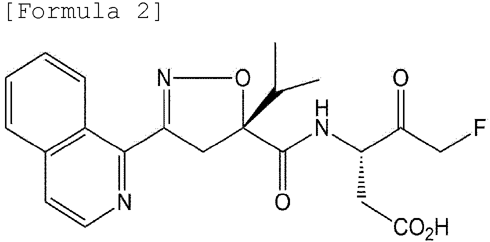

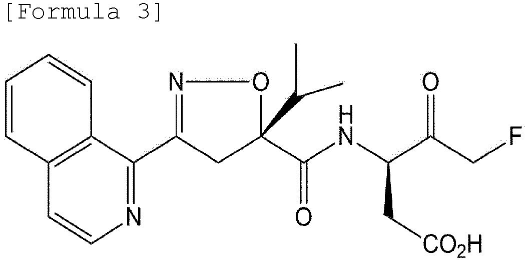

### [Advantageous Effects]

The preparation for injection according to the present invention includes a first formulation, which is a freeze-drying formulation, and a second formulation including a reconstitution solution, and the drug is prepared by mixing the first formulation and the second formulation immediately before administration to a patient, and then the drug is administered to the patient within 30 minutes. Therefore, a preparation for injection with a high content of Active-API can be provided.

In addition, it is possible to provide a freeze-drying preparation for injection with high stability of active pharmaceutical ingredients (API) and long-term storage.

### [Description of Drawings]

Fig. 1 is a diagram showing the results of the XRD pattern measurement of the API freeze-drying formulations manufactured in Examples 9 to 12.
Fig. 2 is a diagram showing the results of the TGA measurement of the freeze-drying formulations manufactured in Examples 9 and 10 and DMSO.
Fig. 3 is a diagram showing (a) the result of the TGA measurement of the freeze-drying formulation manufactured in Example 14, and (b) the result of the DSC measurement of the freeze-drying formulation manufactured in Example 14.
Fig. 4 is a diagram showing (a) the results of the TGA measurement of the freeze-drying formulations manufactured in Examples 17 to 20, and (b) the result of the DSC measurement of the freeze-drying formulations manufactured in Examples 17 to 20.

### [Best Mode]

Hereinafter, the present invention will be described in detail.

All technical terms used in the present invention, unless defined otherwise, are used in the same meaning as commonly understood by those of ordinary skill in the art related to the present invention. In addition, although preferred methods or samples are described in this specification, those similar or equivalent thereto are also included in the scope of the present invention. The contents of all publications incorporated by reference herein are hereby incorporated by reference in their entirety.

Since the compound of Formula 1 of the present invention is hardly soluble in water, the solubility in solvents of various compositions was analyzed to find a solvent capable of dissolving the compound of Formula 1 in a high content.

As a result, it was confirmed that the compound of Formula 1 dissolves well in a basic solvent having a pH of 9.0 or higher, a strong acidic solvent having a pH of 1 or lower or a specific solvent that can be freeze-dried. Further, it was confirmed that the compound of Formula 1 has the property of reducing the pH of a solution while being dissolved in a solvent.

In addition, it was found that when the compound of Formula 1 is dissolved in an aqueous solvent, the lactone ring is broken and converted into the compound of Formula 2 below. At this time, it was found that the compound of Formula 2 is in an active form, but the compound of Formula 2 is rapidly irreversibly converted into the compound of Formula 3, which is a structural isomer, in an aqueous solution, and unlike the compound of Formula 2, the compound of Formula 3 is in an inactive form that does not show the caspase inhibitory activity.

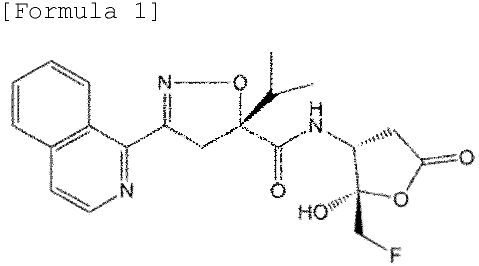

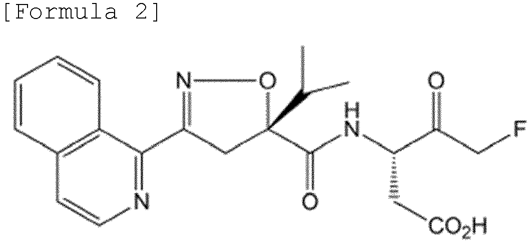

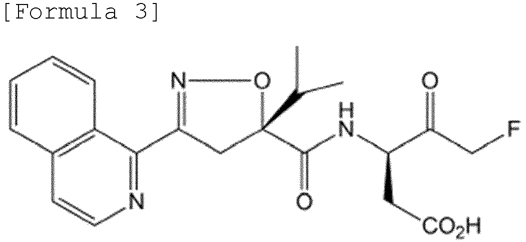

In addition, as a result of confirming the conversion rate from the compound of Formula 2 to the compound of Formula 3 according to the pH of the solvent, it was confirmed that the compound of Formula 3 was hardly produced in an acidic solvent, but the compound of Formula 3 was rapidly produced in a neutral or more basic solvent.

Therefore, it was found that the compound of Formula 1 was well soluble in a basic solvent with high pH, but the active form of the dissolved compound was rapidly changed to the inactive form in a basic solvent. This mechanism lowers the purity of the active ingredient in a preparation for injection using the compound of Formula 1 as an API, so there is a problem of affecting drug efficacy, and since the compound of Formula 1 lowers the pH of the solution as it dissolves, it is difficult to adjust the pH when preparing a final drug for subject administration.

Therefore, as a result of studying various preparation for injection, the present inventors completed a freeze-drying preparation for injection including high-purity API with improved long-term storage stability by dissolving the compound of Formula 1 in a strong acidic solvent of pH 1 or lower or an organic solvent that can be freeze-dried and then freeze-drying thereof.

### Method for manufacturing preparation for injection

According to one embodiment of the present invention, the method for manufacturing a preparation for injection includes the following steps of:
(S1) preparing a first solution by dissolving the compound of Formula 1 below in a solvent including sugars; 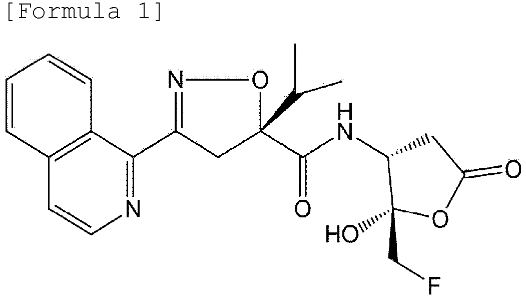
(S2) preparing a first formulation in the powder form by freeze-drying the first solution; and
(S3) preparing a second formulation that is a reconstitution solution including a phosphate buffer, a surfactant, a pH adjusting agent, or a combination thereof.

In one embodiment of the present invention, the method for manufacturing a preparation for injection may include a step of (S1) preparing a first solution by dissolving the compound of Formula 1 below in a solvent including sugars:

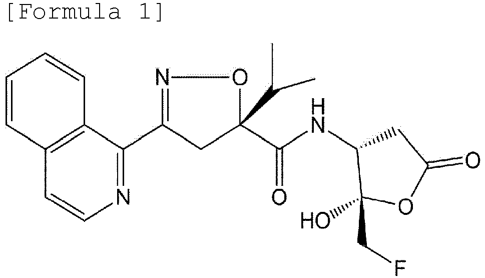

The solvent may include a composition of a solvent capable of dissolving the compound of Formula 1.

The solvent may be a solvent having a pH of 1 or lower. For example, it may be any one selected from the group consisting of hydrochloric acid, perchloric acid, nitric acid and sulfuric acid, but not limited thereto.

In addition, the solvent may be dimethyl sulfoxide or tert-butyl alcohol.

When the solvent is tert-butyl alcohol, the solvent may further include distilled water (DW). The tert-butyl alcohol and distilled water may be mixed at a ratio of 1:1 to 10:1. For example, the compound of Formula 1 may be dissolved in a solvent in which the tert-butyl alcohol and distilled water are mixed at a ratio of 1:1, 2:1, 3:1, 4:1, 5:1, 6:1, 7:1, 8:1, 9:1 or 10:1.

The solvent may include sugars. The sugars may improve the dissolution stability of the compound of Formula 1 and affect the formation of a stable and uniform cake during freeze-drying.

Specifically, the sugars may be at least one selected from the group consisting of mannitol, lactose, sucrose, trehalose and sorbitol, but not limited thereto.

The content of the sugars may be 1 to 10%(w/w) based on the solvent.

In one embodiment of the present invention, the method for manufacturing a preparation for injection may comprise a step of (S2) preparing a first formulation in the powder form by freeze-drying the first solution.

As used herein, the term "freeze-drying" refers to a process in which a material to be dried is first frozen, and then ice or a frozen solvent is removed by sublimation in a vacuum environment.

The freeze-drying may be performed by freezing at -80°C for 3 to 5 hours and then drying at 10 to 20 Pa for 10 to 50 hours.

The first formulation may be in the form of powder, dry powder or lyophile. The first formulation can be stored for a long period of time and is easy to formulate as an injection. In addition, it does not harm the human body at all because there are no additives that may cause side effects.

In one embodiment of the present invention, the method for manufacturing a preparation for injection may comprise a step of (S3) preparing a second formulation that is a reconstitution solution including a phosphate buffer, a surfactant, a pH adjusting agent, or a combination thereof.

As used herein, the term "reconstitution" means that the solid state is returned to the liquid state by adding water or a solution to dry powder such as freeze-dried powder, and the reconstitution solution according to the present invention refers to a solution for preparing a liquid state by adding to the first formulation in powder form.

The second formulation may include a reconstitution medium including distilled water, sterile purified water, water for injection, physiological saline, or a combination thereof, and may include a reconstitution solution further including a phosphate buffer, a surfactant, a pH adjusting agent, or a combination thereof in the reconstitution medium.

The preparation for injection of the present invention may include a buffer in the second formulation. The buffer may be a conventional buffer that can be used for a preparation for injection, and specific examples include phosphate buffer, Tris buffer and MES buffer, but are not limited to. When using the phosphate buffer, it may be selected from the group consisting of sodium phosphate dibasic acid, sodium phosphate monobasic acid, potassium phosphate dibasic acid, potassium phosphate monobasic acid, and mixtures thereof. When using the Tris buffer, TRIZMA^{®} buffer can be used.

The concentration of the buffer may be 50 to 150 mM based on the second formulation, specifically, 50 to 150 mM, 50 to 145 mM, 50 to 140 mM, 50 to 135 mM, 50 to 130 mM, 50 to 125 mM or 50 to 120 mM.

The preparation for injection of the present invention may include a surfactant in the second formulation. The surfactant may help dissolve the compound of Formula 1. The surfactant may be a conventional surfactant that can be used for a preparation for injection, and cyclodextrin-based or polysorbate-based surfactants can be used. Specific examples of the polysorbate-based surfactants may be polysorbate 20, polysorbate 40, polysorbate 60, polysorbate 65 or polysorbate 80, but not limited thereto.

The concentration of the surfactant may be 0.1 to 1.0% based on the second formulation based on the second formulation, specifically, 0.1 to 1.0%, 0.2 to 1.0%, 0.3 to 1.0%, 0.4 to 1.0%, 0.1 to 0.9%, 0.2 to 0.9%, 0.3 to 0.9% or 0.4 to 0.9%.

The preparation for injection of the present invention may include a pH adjusting agent in the second formulation. The pH adjusting agent may be NaOH.

The concentration of NaOH may be 10 to 30 mM based on the second formulation, specifically 10 to 30 mM, 11 to 30 mM, 12 to 30 mM, 13 to 30 mM, 14 to 30 mM, 15 to 30 mM, 10 to 29 mM, 11 to 29 mM, 12 to 29 mM, 13 to 29 mM, 14 to 29 mM, 15 to 29 mM, 10 to 28 mM, 11 to 28 mM, 12 to 28 mM, 13 to 28 mM, 14 to 28 mM, 15 to 28 mM, 10 to 27 mM, 11 to 27 mM, 12 to 27 mM, 13 to 27 mM, 14 to 27 mM, 15 to 27 mM, 10 to 26 mM, 11 to 26 mM, 12 to 26 mM, 13 to 25 mM, 14 to 25 mM, 15 to 25 mM, 10 to 25 mM, 11 to 25 mM, 12 to 25 mM, 13 to 25 mM, 14 to 25 mM or 15 to 25 mM.

### Preparation for injection

According to one embodiment of the present invention, the preparation for injection may comprise a first formulation in the powder form including the compound of Formula 1 below and sugar; and a second formulation that is a reconstitution solution including a phosphate buffer, a surfactant, a pH adjusting agent or a combination thereof:

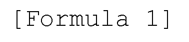

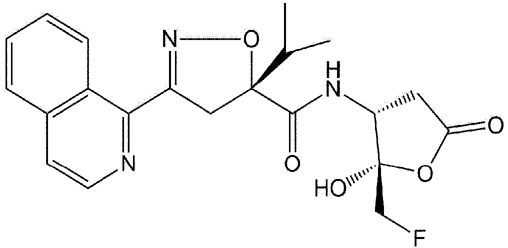

The preparation for injection of the present invention may include sugars in the first formulation. The sugars may improve the dissolution stability of the compound of Formula 1 and affect the formation of a stable and uniform cake during freeze-drying.

Specifically, the sugars may be at least one selected from the group consisting of mannitol, lactose, sucrose, trehalose and sorbitol, but not limited thereto.

The content of the sugars may be 1 to 10%(w/w) based on the solvent.

The first formulation may be in the form of powder, dry powder or lyophilisate. The first formulation can be stored for a long period of time and is easy to formulate as an injection. Further, it does not harm the human body at all because there are no additives that may cause side effects.

The second formulation may include a reconstitution medium including distilled water, sterile purified water, water for injection, physiological saline, or a combination thereof, and may include a reconstitution solution further including a phosphate buffer, a surfactant, a pH adjusting agent or a combination thereof in the reconstitution medium.

The preparation for injection of the present invention may contain a buffer in the second formulation. The buffer may be a conventional buffer that can be used for a preparation for injection, and specific examples include, but are not limited to, phosphate buffer, Tris buffer and MES buffer. When using the phosphate buffer, it may be selected from the group consisting of sodium phosphate dibasic acid, sodium phosphate monobasic acid, potassium phosphate dibasic acid, potassium phosphate monobasic acid, and mixtures thereof. When using the Tris buffer, TRIZMA^{®} buffer can be used.

The concentration of the buffer may be 50 to 150 mM based on the second formulation, specifically, 50 to 150 mM, 50 to 145 mM, 50 to 140 mM, 50 to 135 mM, 50 to 130 mM, 50 to 125 mM or 50 to 120 mM.

The preparation for injection of the present invention may contain a surfactant in the second formulation. The surfactant may help dissolve the compound of Formula 1. The surfactant may be a conventional surfactant that can be used for a preparation for injection, and cyclodextrin-based or polysorbate-based surfactants can be used. Specific examples of the polysorbate-based surfactants may be polysorbate 20, polysorbate 40, polysorbate 60, polysorbate 65 or polysorbate 80, but not limited thereto.

The concentration of the surfactant may be 0.1 to 1.0% based on the second formulation based on the second formulation, specifically, 0.1 to 1.0%, 0.2 to 1.0%, 0.3 to 1.0%, 0.4 to 1.0%, 0.1 to 0.9%, 0.2 to 0.9%, 0.3 to 0.9% or 0.4 to 0.9%.

The preparation for injection of the present invention may contain a pH adjusting agent in the second formulation. The pH adjusting agent may be NaOH.

The concentration of NaOH may be 10 to 30 mM based on the second formulation, specifically 10 to 30 mM, 11 to 30 mM, 12 to 30 mM, 13 to 30 mM, 14 to 30 mM, 15 to 30 mM, 10 to 29 mM, 11 to 29 mM, 12 to 29 mM, 13 to 29 mM, 14 to 29 mM, 15 to 29 mM, 10 to 28 mM, 11 to 28 mM, 12 to 28 mM, 13 to 28 mM, 14 to 28 mM, 15 to 28 mM, 10 to 27 mM, 11 to 27 mM, 12 to 27 mM, 13 to 27 mM, 14 to 27 mM, 15 to 27 mM, 10 to 26 mM, 11 to 26 mM, 12 to 26 mM, 13 to 25 mM, 14 to 25 mM, 15 to 25 mM, 10 to 25 mM, 11 to 25 mM, 12 to 25 mM, 13 to 25 mM, 14 to 25 mM or 15 to 25 mM.

The pH of the solution after reconstitution by mixing the first formulation and the second formulation according to one embodiment of the present invention can be 6.0 to 7.0. If the pH of the solution after the reconstitution is out of the range of 6.0 to 7.0, the patient may feel pain when administering the preparation for injection to the patient, and thus may be unsuitable for use as a preparation for injection.

In the preparation for injection according to one embodiment of the present invention, the osmotic pressure of the solution after reconstitution by mixing the first formulation and the second formulation may be 350 mOsm/kg to 500 mOsm/kg. If the osmotic pressure of the solution after the reconstitution is out of the range of 350 mOsm/kg to 500 mOsm/kg, the patient may feel pain when administering the preparation for injection to the patient, and thus may be unsuitable for use as a preparation for injection.

In the preparation for injection according to one embodiment of the present invention, the amount of the compound of Formula 1 may vary depending on the condition of the patient to be administered, the degree of treatment desired, and the like.

The preparation for injection may include the compound of Formula 1 in an amount of 1 to 20 mg/ml based on the solution after reconstitution by mixing the first formulation and the second formulation. Specifically, the content of the compound of Formula 1 may be 1 to 20 mg/ml, 5 to 20 mg/ml, 10 to 20 mg/ml, 1 to 15 mg/ml, 5 to 15 mg/ml, 10 to 15 mg/ml, 5 mg/ml, 10 mg/ml, 15 mg/ml or 20 mg/ml based on the solution after reconstitution, which may be preferred as a single dose.

When the content of the compound of Formula 1 is at a low concentration of less than 1 mg/ml, a large amount of the injection solution is injected to exhibit a sufficient therapeutic effect, which may cause difficulties in administering to the affected area of the patient, and when the content of the compound of Formula 1 is a high concentration of more than 20 mg/ml, the API may precipitate when the solvent is dissolved or cause pain when administered to the patient's affected area.

In the preparation for injection according to one embodiment of the present invention, the first formulation including the compound of Formula 1 may be mixed with the second formulation to obtain a transparent state in which the compound of Formula 1 is completely dissolved.

The preparation for injection according to one embodiment of the present invention can be used by reconstituting the first formulation including the compound of Formula 1 with the second formulation including the reconstitution solution immediately before administration to the patient and the administering the solution to the affected area of the patient after the reconstitution.

The preparation for injection according to one embodiment of the present invention is used by mixing the first formulation and the second formulation immediately before administration to the patient, so it may not additionally contain excipients for preservation such as antioxidants and preservatives, but in the case of the second formulation including the reconstitution solution, a preservative to prevent secondary contamination may be included.

The present invention provides a method for treating or preventing caspase-related diseases using the preparation for injection of the present invention.

The "treating" means stopping or delaying the progression of the disease when used in a subject showing symptoms of disease, and the "preventing" means stopping or delaying the signs of a disease when used in a high-risk subject who does not show symptoms of disease.

The "caspase-related disease" of the present invention refers to a disease that can be treated or prevented by inhibiting caspase, for example, osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes and gastric ulcer, but is not limited thereto the above disease.

The preparation for injection of the present invention can be administered to a subject in need of treatment or prevention of a caspase-associated disease to treat or prevent the disease in the subject.

The term "administration" or "administering" as used herein refers to a method of administering by injection a dose of the preparation for injection of the present invention to a vertebrate including mammals, birds, fish or amphibians or non-vertebrate animal. Preferred administration methods may be selected from conventional injection methods such as intravascular injection, subcutaneous injection, transdermal injection, intramuscular injection, spinal injection, and intradermal injection, depending on the type of disease to be treated, the site of the disease, and the severity of the disease. In particular, injection into the joint cavity can be selected for diseases related to bone joints such as osteoarthritis, rheumatoid arthritis, degenerative arthritis, or destructive bone disorders.

In the case of injection into the joint cavity using the preparation for injection of the present invention, administration may be guided using an imaging method using ultrasound or the like, or a local anesthetic may be administered in advance or in combination.

The term "subject" as used herein refers to a human or non-human mammal, such as a dog, cat, mouse, rat, cow, sheep, pig, goat, non-human ape, or bird. In some embodiments, the subject is a human.

The preparation for injection of the present invention is administered to the subject within 30 minutes after making a mixed solution by mixing the first solution and the second solution. Therefore, the preparation for injection of the present invention is intended for single-dose for singe patient infusion or administration.

In addition, the preparation for injection of the present invention may be additionally administered in combination with drugs such as analgesics, anti-inflammatory drugs, and steroids. Specifically, it can be administered in combination with drugs such as non-steroidal anti-inflammatory drugs (NSAIDs) selected from ibuprofen, naproxen, aspirin, acetaminophen, indomethacin, diclofenac taken orally or at the site of the lesion, meloxicam, celecoxib, piroxicam, etodolac, nabumetone, lumiracoxib, valdecoxib, etoricoxib, parecoxib, fenoprofen, oxaprozin, mefenamic acid, diflunisal, fluvirofen and ketoprofen; corticoid drugs such as prednisone, methylprednisolone; narcotic pain relievers such as codeine, fentanyl, morphine and meperidine; or injection of hyaluronic acid derivative.

The present invention provides a kit including the preparation for injection of the present invention. In one embodiment of the present invention, the kit may include a preparation for injection including the first formulation in powder form including the compound of Formula 1 of the present invention, and the second formulation including a reconstitution solution; an administration system such as one or more syringes for administering the agent; and instructions for using the kit, including how to mix the first formulation and the second formulation, and directions for treating patients.

In one embodiment of the present invention, the kit may include a label stating that the formulation and contents as described herein are administered to a patient suffering from a disease associated with bone joints such as osteoarthritis, rheumatoid arthritis, degenerative arthritis and destructive bone disorder. Herein, selecting a dosage suitable for the patient is within the knowledge of a person skilled in the art in consideration of the patient's symptoms, age, etc.

Unless otherwise indicated, all numbers used in the specification and claims are to be understood as being modified in all instances by the term "about," whether or not so stated. It should also be understood that the precise numerical values used in the specification and claims form additional embodiments of the disclosure. Efforts have been made to ensure the accuracy of the numerical values disclosed in the Examples. Any measured numerical value, however, can inherently contain certain errors resulting from the standard deviation found in its respective measuring technique.

### Preparation Example

(R)-N-((2S,3S)-2-(fluoromethyl)-2-hydroxy-5-oxotetrahydrofuran-3-yl)-5-isopropyl-3-(isoquinolin-1-yl)-4,5-dihydroisoxazol-5-carboxamide) [(R)-N-((2S,3S)-2-(fluoremethyl)-2-hydroxy-5-oxotetrahydrofuran-3-yl)-5-isopropyl-3-(isoquinoline-1-yl)-4,5-dihydroisooxazol-5-carboxamide), a compound of Formula 1 used as API in the present invention (hereinafter referred to as "API") was prepared according to KR 10-0774999.

### Test Example 1: Preparation of API freeze-drying formulation (Examples 1 to 8)

API was dissolved in the composition of the solvent shown in Table 1 below to prepare an API mixture solution having a concentration of 0.5 mg/ml, and then freeze-dried. The freeze-drying was performed by freezing at -80°C for 6 hours and then drying at 10 to 20 Pa for 18 hours. In addition, after freeze-drying, purified water was used to reconstitute the formulation with a well-formed cake. Then, after reconstitution, the pH, osmotic pressure and API purity of the solution were measured, and the results are shown in Table 2 below.

**[Table 1]**

| **Example** | **Solvent** | **API Conc. (mg/ml)** | **Freeze-drying Suitability** |
|---|---|---|---|
| **Example 1** | 0.1 M HCl | 0.5 | X |
| **Example 2** | 0.1 M HCl:PBS=1:1 | 0.5 | X |
| **Example 3** | 0.1 M HCl | 0.5 | O |
| | 5 % mannitol | | |
| **Example 4** | 0.1 M HCl:PBS=1:1 | 0.5 | O |
| | 5 % mannitol | | |
| **Example 5** | DMSO | 0.5 | X |
| **Example 6** | DMSO:PBS=1:1 | 0.5 | X |
| **Example 7** | DMSO | 0.5 | O |
| | 5 % mannitol | | |
| **Example 8** | DMSO:PBS=1:1 | 0.5 | O |
| | 5 % mannitol | | |

**[Table 2]**

| **Example** | **Reconstitution Solution Composition** | **Reconstitution Time** | **Solution after Reconstitution** | | |
|---|---|---|---|---|---|
| | | | **pH** | **Osmotic Pressure (mOsm/kg)** | **API Purity (%)** |
| **Example 1** | - | - | - | - | - |
| **Example 2** | - | - | - | - | - |
| **Example 3** | Purified water | < 30 sec | | 303 | 100 |
| **Example 4** | Purified water | < 30 sec | | 434 | 100 |
| **Example 5** | - | - | - | - | - |
| **Example 6** | - | - | - | - | - |
| **Example 7** | Purified water | < 30 sec | 3.87 | 346 | 95.40 |
| **Example 8** | Purified water | < 30 sec | 5.79 | 495 | 77.60 |

From the results of Tables 1 and 2 above, it was confirmed that in Examples 3, 4, 7 and 8 in which mannitol was contained in the solvent, white cakes were formed after freeze-drying, confirming that freeze-drying was successful. On the other hand, in Examples 1, 2, 5 and 6 in which mannitol was not contained in the solvent, cakes were not formed after freeze-drying, confirming that the formulations not suitable for use as freeze-drying formulations. In addition, the osmotic pressure of Example 3 was 303 mOsm/kg, whereas the osmotic pressure of Example 4 was 434 mOsm/kg, indicating that the osmotic pressure increased due to phosphate-buffer saline (PBS) in Example 4.

The purity of the API in the solution after reconstitution of Examples 3 and 4 was all 100%, confirming that the API was maintained very stable in strong acid.

It was confirmed that Examples 7 and 8 including DMSO, an organic solvent capable of freeze-drying, were suitable for use as freeze-dried formulations.

The pH of the solution after reconstitution of Example 7 was 3.87, whereas the pH of the solution after reconstitution of Example 8 was 5.79. It was confirmed that the pH of Example 8 was close to neutral due to PBS.

In addition, the osmotic pressure of Example 7 was 346 mOsm/kg, whereas the osmotic pressure of Example 8 was 495 mOsm/kg, indicating that the osmotic pressure increased due to PBS in Example 8.

It is assumed that Examples 7 and 8 contain moisture due to the hygroscopicity of DMSO, which reduces the purity during freeze-drying. In particular, Example 8 contained 50% of PBS, and the purity decreased more rapidly.

### Test Example 2: Preparation of API freeze-drying formulation (Examples 9 to 12)

API was dissolved in the composition of the solvent shown in Table 3 below to prepare an API mixture solution having a concentration of 0.5 mg/ml or 5.0 mg/ml, and then freeze-dried. The freeze-drying was performed by freezing at -80°C for 4 hours and then drying at 10 to 20 Pa for 18 hours.

**[Table 3]**

| **Example** | **Solvent** | **API Conc. (mg/ml)** |
|---|---|---|
| **Example 9** | DMSO | 5.0 |
| | 2.5 % mannitol | |
| **Example 10** | DMSO | 0.5 |
| | 2.5 % mannitol | |
| **Example 11** | 0.1 M HCl | 0.5 |
| | 5.0 % mannitol | |
| **Example 12** | 0.1 M HCl | 0.5 |
| | 2.5 % mannitol | |

The freeze-dried formulations prepared in Examples 9 to 12 were reconstituted, and the purity of the API after reconstitution and the pH and osmotic pressure of the solution after reconstitution were measured. The results are shown in Table 4 below. The solution used for reconstitution of the freeze-dried formulations prepared in Examples 9 to 12 above include 50 mM sodium phosphate buffer (PB) or 0.1 M sodium hydroxide (NaOH). The sodium phosphate buffer was prepared at pH 7.4 by mixing sodium phosphate dibasic acid heptahydrate and sodium phosphate monobasic monohydrate.

**[Table 4]**

| **Example** | **Reconstitu tion Solution Compositio n** | **Reconstitu -tion Time** | **Purity after Reconstitution (%)** | | **Solution after Reconstitution** | |
|---|---|---|---|---|---|---|
| | | | **0 min** | **30 min** | **pH** | **Osmotic Pressure (mOsm/kg)** |
| **Example 9** | 50 mM PB 0.98 ml | 1 min | 96.4 | 94.7 | 6.92 | 311 |
| **Example 10** | 50 mM PB 0.98 ml | 1 min | 95.8 | 93.4 | 7.35 | 266 |
| **Example 11** | 0.1 M NaOH 0.97 ml | < 30 sec | 48.2 | - | 12.54 | 410 |
| **Example 12** | 0.1 M NaOH 0.98 ml | < 30 sec | 41.4 | - | 12.66 | 288 |

From the results in Tables 3 and 4, the freeze-dried formulations prepared in Examples 9, 10, and 12 with a mannitol content of 2.5% formed less cake than the freeze-dried formulations prepared in Example 11 with a mannitol content of 5.0% and did not have a uniform shape. The freeze-dried formulations prepared in Examples 9 and 10 were reconstituted using a phosphate buffer, and all were well dissolved within 1 minute, and the pH of the solution after reconstitution became neutral at about 7.0. After reconstitution of Example 9, the purity of the API in the solution was measured to be 94.7% after 30 minutes of reconstitution. This is because the pH of the solution after reconstitution was slightly lower than that of Example 10, so the purity of the API was slightly higher.

In the freeze-dried formulations prepared in Examples 11 and 12, it was observed that the pH of the solution after reconstitution was about 12 or higher, and the purity of the API in the solution after reconstitution was less than 50%. In Example 11, in which the mannitol content was 5.0%, the osmotic pressure of the solution after reconstitution was 410 mOsm/kg, whereas in Example 12, in which the mannitol content was 2.5%, the osmotic pressure of the solution after reconstitution was 288 mOsm/kg. It is confirmed that the content of mannitol is a factor affecting the osmotic pressure of the solution after reconstitution.

### Test Example 3: XRD pattern analysis of API freeze-drying formulation (Examples 9 to 12)

XRD pattern was measured under the following measurement conditions of the freeze-drying formulations prepared in Examples 9 to 12 above:
- Voltage: 45 kV, Current: 40 mA, Scan range: 4° to 40° (2θ).

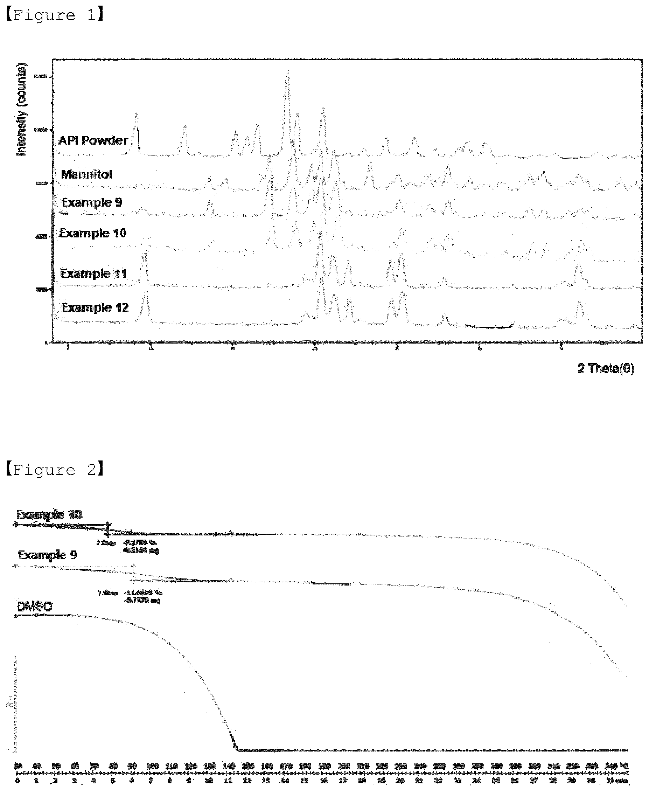

Fig. 1 is the results of the XRD pattern measurement of the API freeze-drying formulations manufactured in Examples 9 to 12.

In Examples 9 to 12, which are API freeze-drying formulations, mannitol peaks were observed, but API powder peaks were not observed (Fig. 1). Through this, it was found that the API was well dissolved in the solution and was well freeze-dried to change into an amorphous API.

In Examples 11 and 12, since some mannitol peaks do not exist or the intensity is very low, it is possible that mannitol has also partially changed into an amorphous form.

### Test Example 4: Determination of residual DMSO in API freeze-drying formulation (Examples 9 to 12)

TGA of the freeze-drying formulations prepared in Examples 9 and 10 was measured under conditions of a temperature range of 30 to 350°C and a heating rate of 10°C/min.

Fig. 2 is the results of the TGA measurement of the freeze-drying formulations manufactured in Examples 9 and 10 and DMSO.

In Fig 2, in the DMSO graph, the weight decreased from about 40 to about 145 °C., which confirmed that the DMSO was evaporating. In addition, the graphs of Examples 9 and 10 also showed weight reduction in the temperature range of 40 to 145°C. Example 9 showed a weight loss of about 11%, and Example 10 showed a weight loss of about 7%, confirming that a considerable amount of residual solvent remained in both Examples 9 and 10. Freeze-drying time may need to be increased to reduce residual DMSO.

### Test Example 5: Preparation of API freeze-drying formulation (Examples 13 to 16)

API was dissolved in the composition of the solvent shown in Table 5 below to prepare an API mixture solution having a concentration of 1.0 mg/ml to 10.0 mg/ml, and then freeze-dried. The freeze-drying was performed by freezing at -80°C for 5 hours and then drying at 10 to 20 Pa for 40 hours.

**[Table 5]**

| **Example** | **Solvent** | **API Conc. (mg/ml)** |
|---|---|---|
| **Example 13** | DMSO | 10.0 |
| | 5.0 % mannitol | |
| **Example 14** | DMSO | 5.0 |
| | 5.0 % mannitol | |
| **Example 15** | 0.5 M HCl | 2.5 |
| | 5.0 % mannitol | |
| **Example 16** | 0.2 M HCl | 1.0 |
| | 5.0 % mannitol | |

The freeze-dried formulations prepared in Examples 13 to 16 were reconstituted, and the purity of the API after reconstitution, and the pH and osmotic pressure of the solution after reconstitution were measured. The results are shown in Table 6 below. The solution used for reconstitution of the freeze-dried formulations prepared in Examples 13 to 16 above include 50 mM sodium phosphate buffer (PB). The sodium phosphate buffer was prepared at pH 7.4 by mixing sodium phosphate dibasic acid heptahydrate and sodium phosphate monobasic monohydrate.

**[Table 6]**

| **Example** | **Reconstit ution Solution Compositi on** | **Reconstitu -tion Time** | **Purity after Reconstitution(%)** | | **Solution after Reconstitution** | |
|---|---|---|---|---|---|---|
| | | | **0 min** | **30 min** | **pH** | **Osmotic Pressure (mOsm/kg)** |
| **Example 13** | 50 mM PB 0.98 ml | 5 min | 97.8 | 96.8 | 6.3 | 479 |
| **Example 14** | 50 mM PB 0.98 ml | 1 min | 97.5 | 96.3 | 6.6 | 765 |
| **Example 15** | 50 mM PB 0.98 ml | X | - | - | 6.3 | 444 |
| **Example 16** | 50 mM PB 0.98 ml | 1 min | 98.2 | 96.8 | 7.1 | 402 |

From the results of Tables 5 and 6, it was difficult to dissolve 2.5 mg/ml API in Example 15, which is a solvent including HCl. This is expected to dissolve the API transparently if the pH is further increased after mixing by changing the composition of the reconstitution solution. The higher the pH of the solution after reconstitution, the greater the decrease in purity 30 minutes after reconstitution compared to the purity immediately after reconstitution.

### Test Example 6: Thermal analysis of API freeze-drying formulation (Example 14)

TGA and DSC of the freeze-drying formulation prepared in Example 14 were measured under conditions of a temperature range of 30 to 350°C and a heating rate of 10°C/min.

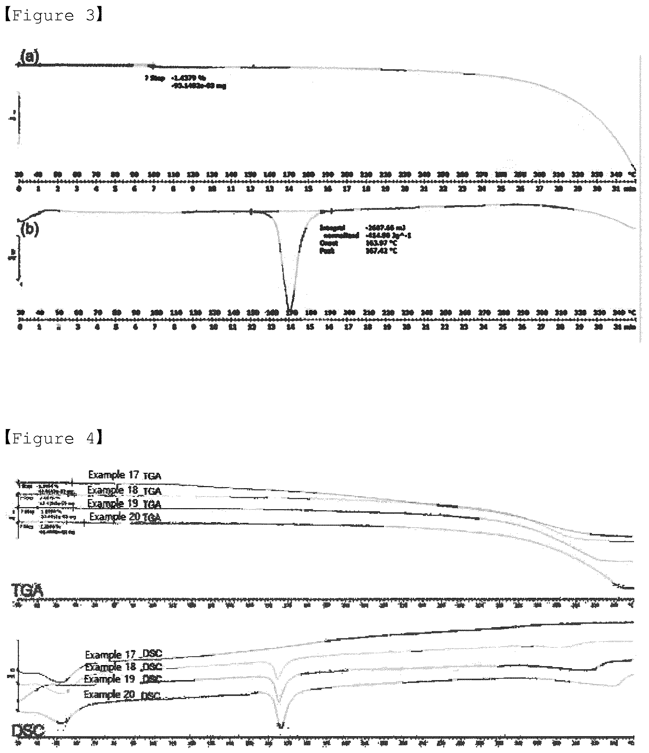

Fig. 3 is (a) the result of the TGA measurement of the freeze-drying formulation manufactured in Example 14, and (b) the result of the DSC measurement of the freeze-drying formulation manufactured in Example 14.

In FIG. 3, (a) the TGA measurement graph showed a weight loss of about 1.4% in the temperature range of about 40 to 145°C at which DMSO evaporated. It was confirmed that in FIG. 2 residual DMSO was greatly reduced compared to Examples 9 and 10. That is, it was confirmed that the amount of residual DMSO decreased when the freeze-drying time was increased.

In FIG. 3, (b) in the DSC measurement graph, an endothermic peak is observed, which can be regarded as the melting point of mannitol(165°C). Therefore, it can be interpreted as meaning that mannitol in the freeze-drying formulation prepared in Example 14 exists in a crystalline form.

### Test Example 7: Preparation of API freeze-drying formulation (Examples 17 to 20)

API was dissolved in the composition of the solvent shown in Table 7 below to prepare an API mixture solution having a concentration of 15.0 mg/ml, and then freeze-dried. The freeze-drying was performed by freezing at -80°C for 3 hours and then drying at 6 Pa for 18 hours.

The content of mannitol was prepared differently according to the ratio of tert-butyl alcohol (TBA) and distilled water (DW) .

Since the freezing point of TBA is 26°C, an API mixture solution was prepared by heating the solvent to 40°C.

**[Table 7]**

| **Example** | **Solvent** | **API Conc. (mg/ml)** |
|---|---|---|
| **Example 17** | TBA | 15 |
| **Example 18** | TBA:DW= 9:1 | 15 |
| | 1 % mannitol | |
| **Example 19** | TBA:DW= 3:1 | 15 |
| | 2.5 % mannitol | |
| **Example 20** | TBA:DW= 1:1 | 15 |
| | 5 % mannitol | |

The properties of the API freeze-dried formulation prepared in Example 17 to 20 were confirmed. The purity and pH of the reconstitution solution of the freeze-dried formulation were measured, and the results are shown in Table 8 below. The solvent used for reconstitution includes 10 mM sodium phosphate buffer or 0.5% polysorbate 80 (Tween 80).

**[Table 8]**

| | **Dissolution of Freeze-drying Formulation in Reconstitution Solution** | | **Solution after Reconstitution** | |
|---|---|---|---|---|
| **Example** | **10 mM PB (0.98 ml)** | **10 mM PB + 0.5 % Tween 80 (0.98 ml)** | **Purity (%)** | **pH** |
| **Example 17** | X | X | 98.69 | 6.97 |
| **Example 18** | X | X | 98.51 | 5.14 |
| **Example 19** | X | X | 98.48 | 5.15 |
| **Example 20** | X | X | 98.19 | 5.15 |

Freeze-dried cakes were normally formed in all of the API freeze-dried formulations prepared in Examples 17 to 20 above. Regarding the freeze-drying formulation, in Example 17, the freeze-dried cake was weak like cotton candy, and a lump with a yellow and sticky texture was formed when reconstituted. It was confirmed that a large amount of API was included in the yellow lump, which was observed as the API was hardly dissolved in the solution after reconstitution, and the pH was also about 7.0.

In Examples 18 to 20 above, the freeze-dried cake in the solution did not melt after reconstitution and became cloudy, and large lumps were present.

In Example 18 to 20 including mannitol, the freeze-dried cake was hard, whereas in Example 17 without mannitol, the freeze-dried cake was not hard. Through this, it was found that a freeze-drying formulation was formed only when a bulking agent such as mannitol was added.

### Test Example 8: Thermal analysis of API freeze-drying formulation (Examples 17 to 20)

TGA and DSC of the freeze-drying formulation prepared in Examples 17 to 20 were measured under conditions of a temperature range of 30 to 350°C and a heating rate of 10°C/min.

Fig. 4 is (a) the results of the TGA measurement of the freeze-drying formulations manufactured in Examples 17 to 20, and (b) the result of the DSC measurement of the freeze-drying formulations manufactured in Examples 17 to 20.

In FIG. 4, (a) in the TGA measurement graph, there was a weight loss due to residual solvent in the 82°C section, which is the boiling point of TBA.

In FIG. 4, (b) in the DSC measurement graph, an endothermic peak at 165°C was observed in Examples 18 to 20, which coincided with the peak of the melting point of mannitol, indicating that mannitol crystals were present in the freeze-dried cake.

### Test Example 9: Analysis of API freeze-drying formulation (Example 19)

The pH, API recovery rate, and purity change for 60 minutes after reconstitution of the reconstitution solution of the formulation prepared in Example 19 were measured, and the results are shown in Table 9 below.

**[Table 9]**

| **Example** | **Reconstitution Solution Composition** | **Reconstit -tion Time** | **pH of Solution after Reconsti tution** | **API Recovery Rate (%)** | **API Purity After Reconstitution (%)** | | |
|---|---|---|---|---|---|---|---|
| | | | | | **2 min** | **30 min** | **60 min** |
| **Example 19** | 120 mM PB | < 2 min | 6.86 | 118.98 | 98.17 | 96.59 | 94.86 |
| | 0.4 % Tween 80 | | | | | | |
| | 25 mM NaOH | | | | | | |

From the results of Table 9, if the pH of the solution after reconstitution is too low, the API is not dissolved, so by adding NaOH to offset the pH that is lowered during the progress of API dissolution, the API was all dissolved.

It was confirmed that the freeze-drying formulation prepared in Example 19 was stable for 60 minutes after reconstitution while securing the purity of API of 94% or more for 60 minutes after reconstitution.

## Claims

1. A method for manufacturing a preparation for injection, comprising:
(S1) preparing a first solution by dissolving the compound of Formula 1 below in a solvent including sugars; 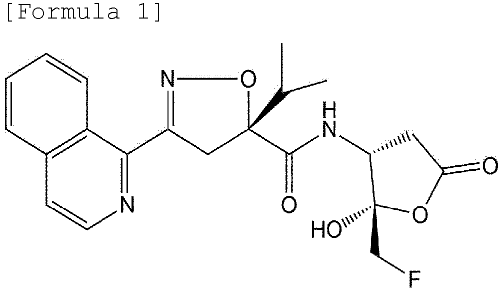
(S2) preparing a first formulation in the powder form by freeze-drying the first solution; and
(S3) preparing a second formulation that is a reconstitution solution including a phosphate buffer, a surfactant, a pH adjusting agent, or a combination thereof.

2. The method for manufacturing a preparation for injection according to claim 1, wherein the pH of the solvent is 1 or lower.

3. The method for manufacturing a preparation for injection according to claim 2, wherein the solvent comprises hydrochloric acid, perchloric acid, nitric acid or sulfuric acid.

4. The method for manufacturing a preparation for injection according to claim 1, wherein the solvent comprises dimethyl sulfoxide or tert-butyl alcohol.

5. The method for manufacturing a preparation for injection according to claim 1, wherein the sugars are at least one selected from the group consisting of mannitol, lactose, sucrose, trehalose and sorbitol.

6. The method for manufacturing a preparation for injection according to claim 1, wherein the content of the sugars is 1 to 10%(w/w) based on the solvent.

7. The method for manufacturing a preparation for injection according to claim 1, wherein the surfactant is at least one selected from the group consisting of polysorbate 20, polysorbate 40, polysorbate 60, polysorbate 65 and polysorbate 80.

8. The method for manufacturing a preparation for injection according to claim 1, wherein the pH adjusting agent contained in the second formulation is NaOH.

9. A preparation for injection, comprising:
a first formulation in the powder form including the compound of Formula 1 and sugars; and
a second formulation that is a reconstitution solution including a phosphate buffer, a surfactant, a pH adjusting agent or a combination thereof:

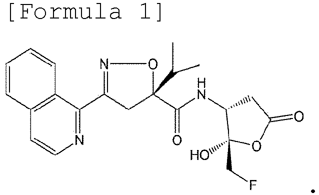

10. The preparation for injection according to claim 9, wherein the sugars are at least one selected from the group consisting of mannitol, lactose, sucrose, trehalose and sorbitol.

11. The preparation for injection according to claim 9, wherein the content of the sugars is 1 to 10%(w/w) based on the solvent.

12. The preparation for injection according to claim 9, wherein the surfactant is at least one selected from the group consisting of polysorbate 20, polysorbate 40, polysorbate 60, polysorbate 65 and polysorbate 80.

13. The preparation for injection according to claim 9, wherein the pH adjusting agent contained in the second formulation is NaOH.

14. The preparation for injection according to claim 9, wherein the content of the compound of Formula 1 is 1 to 20 mg/ml based on the mixed solution of the first formulation and the second formulation.

15. The preparation for injection according to claim 9, wherein the preparation for injection is for treating or preventing a disease selected from the group consisting of osteoarthritis, rheumatoid arthritis, degenerative arthritis, destructive bone disorder, liver disease caused by hepatitis virus, acute hepatitis, liver cirrhosis, brain damage caused by hepatitis virus, human fulminant hepatic failure, sepsis, organ transplant rejection, ischemic heart disease, dementia, stroke, brain damage caused by AIDS, diabetes and gastric ulcer.

16. The preparation for injection according to claim 9, wherein the disease is selected from the group consisting of osteoarthritis, rheumatoid arthritis, degenerative arthritis and destructive bone disorder.

17. The preparation for injection according to claim 9, wherein the preparation for injection is used as a single-dose in a solution state in which the first formulation and the second formulation are mixed.

18. The preparation for injection according to claim 9, wherein the preparation for injection is administered into the joint cavity after mixing the first formulation and the second formulation.

19. The preparation for injection according to claim 9, wherein the preparation for injection is administered into the joint cavity within 30 minutes after mixing the first formulation and the second formulation.

20. The preparation for injection according to claim 9, wherein the preparation for injection is administered once after mixing the first formulation and the second formulation.

21. A preparation for injection, comprising:
a first formulation in the powder form including the compound of Formula 1 below and sugars; and
a second formulation that is a reconstitution solution including a phosphate buffer, a surfactant, a pH adjusting agent or a combination thereof, 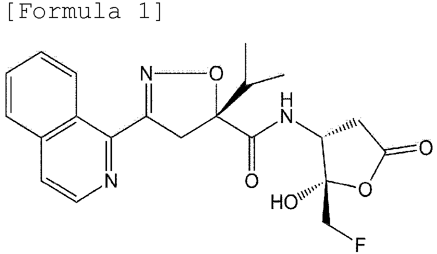
wherein, when the contents of the compounds of Formula 2 and Formula 3 below were measured within 30 minutes after mixing the first formulation and the second formulation, of the total contents of the compounds of Formula 2 and Formula 3, the content of the compound Formula 2 is 94% or more:
